# EUROPÄISCHE PATENTSCHRIFT

(11) **EP 2 637 550 B1**
(45) Veröffentlichungstag und Bekanntmachung des Hinweises auf die Patenterteilung: **11.04.2018**
(21) Anmeldenummer: 11805397.4
(22) Anmeldetag: 11.11.2011
(51) Int. Cl.: A61B 1/06, A61B 1/00

(54) **ENDOSKOP MIT EINSTELLBARER BELEUCHTUNGSRICHTUNG**
ENDOSCOPE WITH ADJUSTABLE ILLUMINATION DIRECTION
ENDOSCOPE DOTÉ D'UN SENS D'ÉCLAIRAGE RÉGLABLE

(30) Priorität: 11.11.2010 DE 102010050931
(43) Veröffentlichungstag der Anmeldung: 18.09.2013
(73) Patentinhaber: Karl Storz SE & Co. KG, 78532 Tuttlingen (DE)
(72) Erfinder: WEIGER, Ulrich, 72414 Rangendingen (DE)
(86) Internationale Anmeldenummer: PCT/EP2011/005682
(87) Internationale Veröffentlichungsnummer: WO 2012/062475

(56) Entgegenhaltungen:
- EP-A1- 1 273 274
- WO-A1-01/39657
- WO-A2-02/096478
- WO-A2-2009/053989
- US-A- 4 398 811
- US-A- 4 718 417
- US-A1- 2010 022 838

## Beschreibung

Die vorliegende Erfindung bezieht sich auf ein Endoskop mit einstellbarer Beleuchtungsrichtung.

Neben Endoskopen für medizinische und nicht-medizinische technische Anwendungen, deren Blickrichtung parallel zur Längsachse des Schafts des Endoskops ist, wurden bereits früh Endoskope mit anderen feststehenden Blickrichtungen entwickelt. Mit der Blickrichtung eines Endoskops ist hier und im Folgenden immer die Richtung vom distalen Ende des Endoskops aus gemeint, in der ein Gegenstand liegt, der in der Mitte des mittels des Endoskops erfassten Bilds erscheint. Bei vielen Anwendungen ist jedoch eine feste Blickrichtung nachteilig. Im ungünstigsten Fall muss beispielsweise während eines medizinischen Eingriffs mehrfach das Endoskop gewechselt werden. In solchen Fällen ist die Verwendung eines Endoskops mit einer in situ einstellbaren bzw. verstellbaren Blickrichtung vorteilhaft.

Die Beobachtung eines Gegenstands in einem Hohlraum mittels eines Endoskops setzt in der Regel eine Beleuchtung des Gegenstands voraus. Dazu weist ein Endoskop beispielsweise Lichtwellenleiter, insbesondere Glasfasern, auf, mittels derer Beleuchtungslicht vom proximalen Ende des Endoskops entlang des Schafts zum distalen Ende des Endoskops übertragen wird. Lichtaustrittsflächen der Lichtwellenleiter am distalen Ende des Endoskops sind so angeordnet und ausgebildet, dass das gesamte Sichtfeld bzw. Blickfeld ausreichend ausgeleuchtet wird.

Bei einem Endoskop mit einstellbarer Blickrichtung wird das Beleuchtungslicht am distalen Ende des Endoskops im einfachsten Fall so verteilt, dass unabhängig von der jeweils eingestellten Blickrichtung das gesamte Sichtfeld ausgeleuchtet ist. Dies hat jedoch eine Reihe von Nachteilen zur Folge. Insbesondere wird Lichtleistung verschwendet, weil ständig unabhängig von der tatsächlich eingestellten Blickrichtung die gesamten Sichtfelder aller einstellbaren Blickrichtungen ausgeleuchtet werden. Bei einer vorbestimmten erwünschten Helligkeit muss somit insgesamt eine deutlich höhere Lichtleistung zur Verfügung gestellt werden als bei einem Endoskop mit feststehender Blickrichtung.

Ein weiterer Nachteil rührt daher, dass Beleuchtungslicht hoher Intensität Gewebe oder andere Gegenstände photothermisch oder photochemisch schädigen kann. Bei einem Endoskop mit feststehender Blickrichtung fällt ein zu geringer Abstand des distalen Endes des Endoskops zu einem Gegenstand zumindest bei Betrachtung des erfassten Bilds in der Regel auf. Bei Verwendung einer Kamera am Endoskop ist auch eine automatische Warnung von Benutzern möglich, wenn die Helligkeit eines erfassten Bilds eine vorbestimmte Schwelle überschreitet. Bei einem Endoskop mit einstellbarer Blickrichtung fällt jedoch ein Teil des Beleuchtungslichts auf Gegenstände, die außerhalb des Sichtfelds liegen. Eine unerwünschte Annäherung des distalen Endes des Endoskops an diese Gegenstände und eine resultierende Bestrahlung dieser Gegenstände mit einer zu hohen Strahlungsleistung fallen deshalb nicht auf.

Ein weiterer Nachteil besteht darin, dass auch Beleuchtungslicht, das außerhalb des Sichtfelds abgestrahlt wird, von Gegenständen oder opaken Medien gestreut oder reflektiert werden kann. Das reflektierte oder gestreute Beleuchtungslicht kann direkt oder indirekt in den Beobachtungs-Strahlengang gelangen. Dadurch können Kontraste und vor allem in dunklen Bildbereichen die Unterscheidbarkeit von Gegenständen verringert werden.

Ein weiterer Nachteil rührt daher, dass die Beleuchtungsstärke bzw. die Intensität des Beleuchtungslichts in der Richtung, in der die Blickrichtung variiert werden kann (auch als vertikale Richtung bezeichnet) im Wesentlichen konstant ist, während sie in der dazu senkrechten Richtung (auch als horizontale Richtung bezeichnet) in der Regel zum Rand des Sichtfelds hin leicht abnimmt. Von Endoskopen mit feststehender Blickrichtung sind Benutzer jedoch in der Regel eine Beleuchtungsstärke gewohnt, die sowohl in horizontaler als auch in vertikaler Richtung zum Rand des Sichtfelds hin leicht abnimmt. Die in vertikaler Richtung konstante Beleuchtungsstärke kann deshalb als irritierend empfunden werden. In der DE 600 15 375 T2 ist eine Anordnung mehrerer Prismen beschrieben. Eines der Prismen ist um eine Achse rotierbar, um Beleuchtungslicht in eine einstellbare Blickrichtung zu werfen. Die Erfinder der vorliegenden Erfindung haben jedoch festgestellt, dass die Lagerung des rotierbaren Prismas in vielen Fällen konstruktiv und in der Herstellung aufwändig ist. Dokument WO 01/39657 A1 offenbart ein Arthoroskop gemäß des Oberbegriffs des Anspruchs 1. Eine Aufgabe der vorliegenden Erfindung besteht darin, ein verbessertes Endoskop mit einstellbarer Beleuchtungsrichtung zu schaffen.

Diese Aufgabe wird durch den Gegenstand des unabhängigen Anspruchs 1 gelöst. Weiterbildungen sind in den abhängigen Ansprüchen angegeben.

Ein Endoskop mit einstellbarer Beleuchtungsrichtung umfasst eine reflektierende Fläche zum Reflektieren von Beleuchtungslicht zur Beleuchtung eines mittels des Endoskops beobachteten Gegenstands, wobei die reflektierende Fläche verschiebbar ist, um die Beleuchtungsrichtung einzustellen.

Das Endoskop ist insbesondere ausgebildet, um die Blickrichtung und die Beleuchtungsrichtung gemeinsam zu schwenken. Die Blickrichtung ist die Richtung, in der ein Gegenstand bezogen auf das distale Ende des Endoskops liegt, der bei Betrachtung durch das Endoskop in der Mitte eines erfassten Bilds erscheint. Die Beleuchtungsrichtung ist die mittlere Richtung bezogen auf das distale Ende des Endoskops, in die das Beleuchtungslicht abgestrahlt wird. Die Beleuchtungsrichtung und die Blickrichtung entsprechen einander insbesondere. Alternativ sind die Blickrichtung und die Beleuchtungsrichtung unabhängig voneinander einstellbar oder die Beleuchtungsrichtung relativ zur Blickrichtung innerhalb vorbestimmter Grenzen variierbar, um beispielsweise bei einer kleinen Gegenstandsweite eine vollständige Ausleuchtung des beobachteten Bereichs zu erreichen.

Die Blickrichtung und die Beleuchtungsrichtung des Endoskops sind insbesondere um eine oder zwei (insbesondere parallele) Schwenkachsen schwenkbar, die senkrecht zur Längsachse des Schafts des Endoskops ist. Die Längsachse des Endoskops ist insbesondere die Längsachse des Schafts. Im Fall eines starren geraden Schafts ist die Längsachse des Schafts die Gerade, auf der die Mittelpunkte der Querschnittsflächen des Schafts liegen. Im Fall eines flexiblen Schafts ist die Längsachse des Endoskops die Längsachse des distalen Endes des Schafts, also die Gerade, auf der Mittelpunkte der Querschnittsflächen des Schafts nahe dessen distalem Ende liegen.

Das Endoskop kann so ausgebildet wein, dass mit einem Verschieben der reflektierenden Fläche ein Schwenken der reflektierenden Fläche einhergeht. Eine Verschiebbarkeit der reflektierenden Fläche im Sinne dieser Anmeldung umfasst jedoch keine einfache Schwenkbarkeit um eine am distalen Ende des Endoskops in dessen Schaft liegende Achse.

Ein Endoskop, wie es hier beschrieben ist, umfasst insbesondere eine Linearführung zum Führen der verschiebbaren reflektierenden Fläche entlang eines geraden oder gekrümmten Pfads.

Eine verschiebbare reflektierende Fläche kann durch eine Linearführung präzise geführt sein. Eine gerade oder gekrümmte Linearführung kann beispielsweise mit einer oder mehreren Nuten oder Stegen mit geringem Aufwand und auch stark miniaturisiert herstellbar sein. Die Linearführung kann so ausgeführt werden, dass sie lediglich einen geringen Bauraum in Anspruch nimmt. Alternativ oder zusätzlich kann die verschiebbare reflektierende Fläche mittels eines oder mehrerer Pleuel, Kupplungsstangen, Gleitsteine oder Kreuzköpfe oder mittels einer oder mehrerer anderer Koppeln geführt sein, um entlang eines geraden oder gekrümmten Pfads verschiebbar zu sein.

Insbesondere wenn eine optische Einrichtung am distalen Ende des Endoskops zum Einstellen der Blickrichtung mittels einer Schub- oder Zugstange im Schaft des Endoskops betätigt wird, kann die Bewegung dieser Schub- bzw. Zugstange mit geringem mechanischem Aufwand auf die verschiebbare reflektierende Fläche übertragen werden. Im einfachsten Fall ist die verschiebbare reflektierende Fläche unmittelbar mit der Schub- bzw. Zugstange mechanisch gekoppelt. Der beispielsweise zum Übersetzen einer Linearbewegung in eine Rotations- bzw. Schwenkbewegung erforderliche Aufwand eines mechanischen Getriebes kann damit weitgehend oder vollständig entfallen.

Die verschiebbare reflektierende Fläche ist insbesondere gekrümmt. Beispiele für eine gekrümmte Gestalt der verschiebbaren reflektierenden Fläche sind nachfolgend dargestellt.

Bei einem Endoskop, wie es hier beschrieben ist, kann die reflektierende Fläche in einer Richtung verschiebbar sein, die von der Längsachse des Endoskops um nicht mehr als 45 Grad abweicht.

Insbesondere weicht die Richtung, in der die reflektierende Fläche verschiebbar ist, von der Längsachse des Endoskops um nicht mehr als 30 Grad oder nicht mehr als 20 Grad ab oder ist zur Längsachse des Endoskops parallel oder im Wesentlichen parallel. Ein kleiner Winkel zwischen der Richtung, in der die reflektierende Fläche verschiebbar ist, und der Längsachse des Endoskops ermöglicht eine mechanische Ansteuerung mit geringem Aufwand. Insbesondere kann die reflektierende Fläche mit der oben erwähnten Schub- oder Zugstange unmittelbar oder lediglich mittels eines einfachen biegeelastischen Elements gekoppelt sein. Wenn die Richtung, in der die reflektierende Fläche verschiebbar ist, zur Längsachse des Endoskops parallel oder im Wesentlichen parallel (höchstens 10 Grad, insbesondere höchstens 5 Grad) ist, kann die reflektierende Fläche beispielsweise unmittelbar an der Schub- bzw. Zugstange befestigt und mit dieser geführt sein. Eine verstellbare Beleuchtungsrichtung kann so auf besonders einfache und kostengünstige Weise erzielbar sein.

Bei einem Endoskop, wie es hier beschrieben ist, kann die reflektierende Fläche entlang eines geraden Pfads verschiebbar sein.

Die im vorangehenden Absatz genannten Vorteile gelten insbesondere, wenn die reflektierende Fläche entlang eines geraden Pfads verschiebbar ist. Aber auch wenn die reflektierende Fläche entlang eines geraden Pfads verschiebbar ist, der nicht parallel zur Längsachse des Endoskops ist, kann die Verschiebbarkeit entlang eines geraden Pfads vorteilhaft sein. Insbesondere können gerade Schienen, Stege oder Nuten oder anderen Linearführungen einfach herstellbar sein und eine spielarme Führung ermöglichen.

Bei einem Endoskop, wie es hier beschrieben ist, kann alternativ die reflektierende Fläche entlang eines gekrümmten Pfads verschiebbar sein, der einen lokalen Krümmungsmittelpunkt aufweist, der zumindest entweder vom Ort der reflektierenden Fläche an dem gekrümmten Pfad abhängig ist oder außerhalb des Schafts des Endoskops liegt.

Der gekrümmte Pfad kann neben einem oder mehreren gekrümmten Abschnitten einen oder mehrere gerade Abschnitte umfassen. Der gekrümmte Pfad oder ein gekrümmter Abschnitt des Pfads kann kreisbogenförmig sein und damit einen einzigen Krümmungsmittelpunkt aufweisen. Alternativ kann der gekrümmte Pfad oder ein gekrümmter Abschnitt des Pfads eine ortsabhängige Krümmung und einen ortsabhängigen Krümmungsmittelpunkt aufweisen. Insbesondere liegt der gekrümmte Pfad in einer Ebene, die senkrecht zu einer Schwenkachse der Blickrichtung des Endoskops und insbesondere parallel zu allen Blickrichtungen des Endoskops ist.

Ein gekrümmter Pfad kann die Wirkung einer Verschiebung der reflektierenden Fläche auf die Beleuchtungsrichtung verstärken. Ferner kann ein gekrümmter Pfad die Miniaturisierbarkeit fördern. Insbesondere kann durch die Krümmung des Pfads eine Kollision der reflektierenden Fläche mit anderen Einrichtungen am distalen Ende des Endoskops vermieden werden bzw. es kann unter den räumlich beengten Verhältnissen am distalen Ende des Endoskops ein gekrümmter Pfad eine größere Länge aufweisen als ein gerader Pfad.

Ein Endoskops, wie es hier beschrieben ist, kann ausgebildet sein, um Beleuchtungslicht aus einer Richtung auf die verschiebbare reflektierende Fläche zu werfen, die nicht parallel zur Längsachse des Endoskops ist. Wenn das auf die verschiebbare reflektierende Fläche fallende Beleuchtungslicht nicht kollimiert ist, ist die Richtung des Beleuchtungslichts die mittlere Richtung des auf die verschiebbare reflektierende Fläche fallenden Beleuchtungslichtbündels. Die Richtung, aus der das Beleuchtungslicht auf die verschiebbare reflektierende Fläche zu werfen das Endoskop ausgebildet ist, ist insbesondere senkrecht oder im Wesentlichen senkrecht zur Längsachse des Endoskops oder schließt mit dieser einen Winkel von mindestens 45 Grad oder mindestens 60 Grad oder mindestens 75 Grad ein. Insbesondere sind die reflektierende Fläche parallel oder im Wesentlichen parallel zur Längsachse des Endoskops verschiebbar und das Endoskop ausgebildet, um Beleuchtungslicht aus einer Richtung senkrecht zur Längsachse des Endoskops auf die verschiebbare reflektierende Fläche zu werfen.

Ein Endoskop, wie es hier beschrieben ist, kann eine Lichtaustrittsfläche zum Bestrahlen der verschiebbaren reflektierenden Fläche mit Beleuchtungslicht umfassen, wobei die Flächennormale der Lichtaustrittsfläche nicht parallel zur Längsachse des Endoskops und nicht parallel zur einer Schwenkachse der Beleuchtungsrichtung oder zu einer Schwenkachse der Blickrichtung ist.

Die Lichtaustrittsfläche ist insbesondere eine Lichtaustrittsfläche eines Lichtleiters, einer Lichtquelle, eines Prismas oder eines anderen transparenten Körpers zum Lenken oder Umlenken des Beleuchtungslichts. Wenn die Lichtaustrittsfläche gekrümmt ist, ist mit der Flächennormale der Lichtaustrittsfläche insbesondere die mittlere Flächennormale der Lichtaustrittsfläche gemeint. Die Flächennormale der Lichtaustrittsfläche schließt mit der Längsachse des Endoskops insbesondere einen Winkel von mindestens 45 Grad oder von mindestens 60 Grad oder von mindestens 75 Grad ein. Die Flächennormale der Lichtaustrittsfläche schließt mit der Schwenkachse der Beleuchtungsrichtung und/oder mit der Schwenkachse der Blickrichtung einen Winkel ein, der mindestens 45 Grad oder mindestens 60 Grad oder mindestens 75 Grad beträgt. Insbesondere ist die Flächennormale der Lichtaustrittsfläche senkrecht oder im Wesentlichen senkrecht zumindest entweder zur Längsachse des Endoskops oder zur Schwenkachse der Beleuchtungsrichtung oder zur Schwenkachse der Blickrichtung.

Bei einem Endoskop, wie es hier beschrieben ist, kann eine Schnittlinie der verschiebbaren reflektierenden Fläche mit einer Ebene, die zu zwei einstellbaren Beleuchtungsrichtungen des Endoskops parallel ist, konvex gekrümmt sein.

Eine konvexe Krümmung der Schnittlinie kann eine Veränderung der Beleuchtungsrichtung durch ein Verschieben der reflektierenden Fläche in der zu den zwei einstellbaren Beleuchtungsrichtungen parallelen Ebene ermöglichen. In Richtung senkrecht zu der zu den zwei einstellbaren Beleuchtungsrichtungen parallelen Ebene kann die verschiebbare reflektierende Fläche ebenfalls gekrümmt sein. Insbesondere hat die verschiebbare reflektierende Fläche eine negative Gaußsche Krümmung K, weist also die Gestalt einer Sattelfläche auf. Bezogen auf eine Richtung senkrecht zu der zu den zwei einstellbaren Beleuchtungsrichtungen parallelen Ebene, kann die verschiebbare reflektierende Fläche somit eine die Divergenz des Beleuchtungslichts reduzierende oder eine Konvergenz des Beleuchtungslichts erhöhende Wirkung aufweisen. Dadurch kann die Breite (gemessen in einer Richtung senkrecht zu der zu den zwei einstellbaren Beleuchtungsrichtungen parallelen Ebene) eines Austrittsfensters für das Beleuchtungslicht verringert werden.

Alternativ ist die Schnittlinie der verschiebbaren reflektierenden Flächen mit einer Ebene, die zu zwei einstellbaren Beleuchtungsrichtungen des Endoskops parallel ist, konkav gekrümmt. Ferner kann die Schnittlinie abschnittsweise konvex und/oder abschnittsweise konkav gekrümmt sein.

Auch eine konkave Krümmung der Schnittlinie kann ein Einstellen der Beleuchtungsrichtung durch ein Verschieben der reflektierenden Fläche in der zu den zwei einstellbaren Beleuchtungsrichtungen parallelen Ebene ermöglichen.

Bei einem Endoskops, wie es hier beschrieben ist, kann die verschiebbare reflektierende Fläche einen ersten Abschnitt und einen zweiten Abschnitt umfassen, wobei zumindest entweder der erste Abschnitt und der zweite Abschnitt nicht glatt ineinander übergehen oder die Schnittlinien des ersten Abschnitts und des zweiten Abschnitts mit einer Ebene, die zu zwei einstellbaren Beleuchtungsrichtungen des Endoskops parallel ist, entgegengesetzt gekrümmt sind.

Insbesondere sind der erste Abschnitt und der zweite Abschnitt der verschiebbaren reflektierenden Fläche für unterschiedliche Winkelbereiche der Beleuchtungsrichtung vorgesehen. Beispielsweise wird Beleuchtungslicht in Beleuchtungsrichtungen innerhalb eines ersten Winkelbereichs geworfen, wenn es auf den ersten Abschnitt der verschiebbaren reflektierenden Fläche trifft, und in Beleuchtungsrichtungen innerhalb eines zweiten Winkelbereichs geworfen, wenn es auf den zweiten Abschnitt der verschiebbaren reflektierenden Fläche trifft.

Insbesondere schließen der erste Winkelbereich und der zweite Winkelbereich aneinander an oder überlappen (insbesondere geringfügig). Eine Segmentierung der reflektierenden Fläche in mehrere Abschnitte, die nicht glatt ineinander übergehen, oder die entgegengesetzt gekrümmt sind, kann einen größeren Gesamtwinkelbereich der einstellbaren Beleuchtungsrichtungen ermöglichen. Der erste Abschnitt und der zweite Abschnitt gehen nicht glatt ineinander über, wenn zwischen dem ersten Abschnitt und dem zweiten Abschnitt ein Sprung, eine Stufe oder ein Knick vorliegt bzw. wenn die reflektierende Fläche zwischen dem ersten Abschnitt und dem zweiten Abschnitt nicht stetig oder nicht differenzierbar ist.

Bei einem Verfahren zu Einstellen der Beleuchtungsrichtung eines Endoskops, wird eine reflektierende Fläche zum Reflektieren von Beleuchtungslicht zur Beleuchtung eines mittels des Endoskops beobachteten Gegenstands bereitgestellt. Die reflektierende Fläche wird verschoben, um die Beleuchtungsrichtung einzustellen.

Bei dem Verfahren wird die reflektierende Fläche insbesondere durch eine gerade oder gekrümmte Linearführung geführt.

Bei dem Verfahren wird Beleuchtungslicht insbesondere aus einer Richtung auf die reflektierende Fläche geworfen, die nicht parallel zur Längsachse des Endoskops und nicht parallel zumindest entweder zu einer Schwenkachse der Blickrichtung oder zu einer Schwenkachse der Beleuchtungsrichtung ist.

Das Verfahren ist insbesondere mit einem Endoskop, wie es hier beschrieben ist, durchführbar.

### Kurzbeschreibung der Figuren

Nachfolgend werden Ausführungsformen anhand der beigefügten Figuren näher erläutert. Es zeigen:
- Figur 1: eine schematische Darstellung eines Endoskops mit einstellbarer Blickrichtung;
- Figur 2: eine schematische Darstellung des distalen Endes einer Ausführungsform eines Endoskops;
- Figur 3: eine weitere schematische Darstellung des distalen Endes aus Figur 2;
- Figur 4: eine schematische Darstellung des distalen Endes einer weiteren Ausführungsform eines Endoskops;
- Figur 5: eine schematische Darstellung des distalen Endes einer weiteren Ausführungsform eines Endoskops;
- Figur 6: eine schematische Darstellung des distalen Endes einer weiteren Ausführungsform eines Endoskops;
- Figur 7: eine weitere schematische Darstellung des distalen Endes aus Figur 6;
- Figur 8: eine schematische Darstellung des distalen Endes einer weiteren Ausführungsform eines Endoskops
- Figur 9: eine schematische Darstellung des distalen Endes einer weiteren Ausführungsform eines Endoskops;
- Figur 10: eine weitere schematische Darstellung des distalen Endes aus Figur 9;
- Figur 11: eine schematische Darstellung des distalen Endes einer weiteren Ausführungsform eines Endoskops;
- Figur 12: ein schematisches Flussdiagramm eines Verfahrens zum Einstellen der Beleuchtungsrichtung eines Endoskops.

### Beschreibung von Ausführungsformen

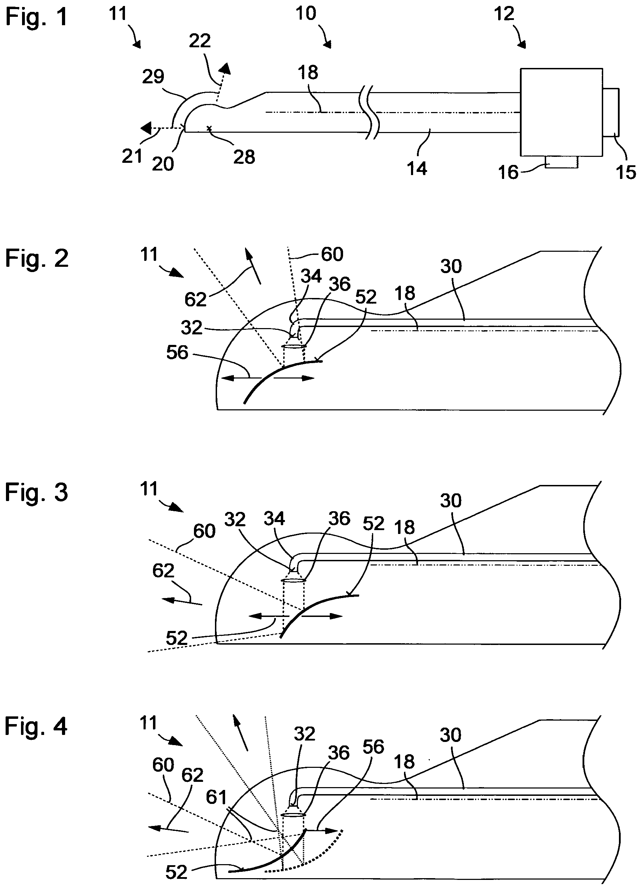

Figur 1 zeigt eine schematische Darstellung eines Endoskops 10 mit einem distalen Ende 11, einem proximalen Ende 12 und einem starren Schaft 14, der sich vom distalen Ende 11 bis zum proximalen Ende 12 erstreckt. Alternativ ist der Schaft 14 flexibel oder teilweise flexibel. Der Querschnitt des Schafts 14 oder zumindest die äußere Kontur des Querschnitts des Schafts 14 ist zwischen dem distalen Ende 11 und dem proximalen Ende 12 konstant oder im Wesentlichen konstant. Insbesondere ist die Kontur des Querschnitts des Schafts 14 kreisförmig oder elliptisch. In diesem Fall ist die in Figur 1 dargestellte Längsachse 18 des Endoskops 10 die Symmetrieachse der Mantelfläche des Schafts 14 zwischen dem distalen Ende 12 und dem proximalen Ende 11. Bei einer zylindrischen Mantelfläche des Schafts 14 ist die Längsachse 18 auch die Menge der Mittelpunkte oder Flächenschwerpunkte der Querschnitte des Schafts 14 zwischen dem distalen Ende 12 und dem proximalen Ende 11.

Am distalen Ende 12 weicht die Gestalt des Schafts 14 von der Zylindersymmetrie ab, wie dies beispielhaft in Figur 1 dargestellt ist. Insbesondere weist der Schaft 14 am distalen Ende 12 eine Öffnung auf, die durch ein transparentes Fensterbauteil mit einer gewölbten Oberfläche 20 verschlossen ist, insbesondere hermetisch dicht verschlossen ist. Die Oberfläche 20 des Fensterbauteils hat beispielsweise die Gestalt eines Ausschnitts eines Kreiszylindermantels, wobei die Symmetrieachse des Kreiszylinders senkrecht zur Längsachse 18 des Endoskops 10 und zur Zeichenebene der Figur 1 ist. Alternativ hat die Oberfläche 20 des transparenten Fensterbauteils die Gestalt eines Ausschnitts einer Kugeloberfläche oder eines rotationssymmetrischen oder nicht rotationssymmetrischen Ellipsoids. Das Fensterbauteil ist Teil des Beobachtungs- und/oder des Beleuchtungsstrahlengangs. Für den Beobachtungsstrahlengang und einen oder mehrere Beleuchtungsstrahlengänge können separate Fensterbauteile vorgesehen sein.

Am distalen Enden 12 des Endoskops 10 sind im Schaft 14 optische Einrichtungen angeordnet, die eine Variation der Blickrichtung zwischen einer ersten extremen Blickrichtung 21 und einer zweiten extremen Blickrichtung 22 ermöglichen und in Figur 1 nicht dargestellt sind. Die Blickrichtung ist jeweils die Richtung bezogen auf das distale Ende 12 des Endoskops 10, in der ein Gegenstand liegt, der in der Mitte eines mittels des Endoskops 10 erfassten Bilds erscheint.

Bei dem in Figur 1 dargestellten Beispiel ist die erste, extreme Blickrichtung 21 parallel oder im Wesentlichen parallel zur Längsachse 18 des Endoskops 10. Zwischen den extremen Blickrichtungen 21, 22 liegt ein Winkelbereich 29, der bei dem dargestellten Beispiel ca. 120° umfasst. Innerhalb dieses Winkelbereichs ist die Blickrichtung des Endoskops 10 insbesondere kontinuierlich verstellbar.

Nachfolgend werden anhand der Figuren 2 bis 7 Einrichtungen zum Einstellen der Beleuchtungsrichtung dargestellt. Die Beleuchtungsrichtung ist insbesondere identisch mit der Blickrichtung oder weicht von dieser nur geringfügig ab. Alternativ können die Beleuchtungsrichtung und die Blickrichtung unabhängig voneinander eingestellt oder eine Winkeldifferenz zwischen ihnen eingestellt werden. Nachfolgend wird der Einfachheit halber nur auf die Beleuchtungsrichtung eingegangen, wobei Beleuchtungsrichtung und Blickrichtung gleichgesetzt werden.

Am proximalen Ende 11 weist das Endoskop 10 eine erste Kupplung 15 zum optischen Koppeln des Endoskops 10 mit einer Kamera oder ein Okular sowie eine zweite Kupplung 16 zum Koppeln des Endoskops 10 mit einer Lichtquelle über ein Lichtleitkabel auf. Von der zweiten Kupplung 16 führen ein oder mehrere Lichtleiter 30 durch den Schaft 14 bis zum distalen Ende 11 des Endoskops 10. Von einer Lichtquelle erzeugtes Beleuchtungslicht kann über ein Lichtleitkabel, die zweite Kupplung 16 und den oder die Lichtleiter 30 zum distalen Ende 11 des Endoskops 10 übertragen werden.

Die Figuren 2 und 3 zeigen schematische Schnittdarstellungen einer Ausführungsform des distalen Endes 11 eines Endoskops, beispielsweise des oben anhand der Figur 1 dargestellten Endoskops 10. Die dargestellte Schnittebene ist parallel zur in Figur 1 gezeigten Längsachse 18 des Endoskops 10. Im Schaft des Endoskops erstreckt sich ein Lichtleiter 30 von der in Figur 1 gezeigten zweiten Kupplung 16 bis zum distalen Ende 11 des Endoskops 10. Am distalen Ende 11 weist der Lichtleiter 30 eine Lichtaustrittsfläche 32 und unmittelbar lichtstromaufwärts der Lichtaustrittsfläche 32 eine Krümmung 34 auf. An der Krümmung 34 ändert der Lichtleiter 30 seine Richtung, bzw. die Richtung, in der er Beleuchtungslicht leitet bzw. überträgt, um ca. 90 Grad.

Lichtstromabwärts der Lichtaustrittsfläche 32 des Lichtleiters 30 und gegenüber der Lichtaustrittsfläche 32 ist eine Linse 36 angeordnet. Die Linse 36 ist bei dem in den Figuren 2 und 3 gezeigten Beispiel eine Sammellinse, die die Divergenz des aus der Lichtaustrittsfläche 32 des Lichtleiters 30 austretenden Beleuchtungslichts reduziert. Insbesondere ist die Line 36 als Kollimator ausgebildet und angeordnet, so dass das Beleuchtungslicht lichtstromabwärts der Linse 36 im Wesentlichen parallel ausgerichtet ist und einen im Wesentlichen konstanten Strahlquerschnitt aufweist. Anstelle einer einzelnen Linse können mehrere Linsen bzw. ein Objektiv, einer oder mehrere Spiegel oder ein Beugungsgitter vorgesehen sein.

Lichtstromabwärts der Linse 36 ist eine verschiebbare gekrümmte reflektierende Fläche 52, insbesondere ein verschiebbarer gekrümmter Spiegel, angeordnet. Die gekrümmte reflektierende Fläche 52 ist in einer Richtung verschiebbar, die in den Figuren 2 und 3 durch Pfeile 56 angedeutet ist. Insbesondere ist die gekrümmte reflektierende Fläche 52 entlang eines geraden Pfads verschiebbar. Insbesondere ist die gekrümmte reflektierende Fläche 52 in einer Richtung 56 parallel zur Längsachse 18 des Endoskops 10 verschiebbar. Alternativ kann die gekrümmte reflektierende Fläche 52 entlang eines geraden Pfads, der nicht parallel zur Längsachse 18 des Endoskops 10 ist, oder entlang eines zumindest abschnittsweise gekrümmten Pfads verschiebbar sein. Ein gekrümmter Pfad, entlang dessen die gekrümmte reflektierende Fläche 52 verschiebbar ist, weist insbesondere einen ortsabhängigen Krümmungsmittelpunkt und/oder zumindest einen Krümmungsmittelpunkt, der außerhalb der Schwenkachse 28 der Blickrichtung 21, 22 liegt, auf.

In den Figuren 2 und 3 ist die verschiebbare gekrümmte reflektierende Fläche 52 in zwei verschiedenen Positionen dargestellt. Durch gestrichelte Linien 60 sind in beiden Figuren 2 und 3 Ränder bzw. Konturen von Beleuchtungs-Lichtkegeln, die von der Lichtaustrittsfläche 32 des Lichtleiters 30 ausgehen, angedeutet. Obwohl Lichtkegel schon aufgrund von Beugungseffekten nicht scharf begrenzt sein können, deuten die gestrichelten Linien 60 die Ausbreitung des Beleuchtungslichts für Fachleute erkennbar an.

Im Vergleich der Figuren 2 und 3 ist erkennbar, dass von der Lichtaustrittsfläche 32 ausgehendes Beleuchtungslicht aufgrund der Krümmung der reflektierenden Fläche 52 abhängig von der Position der gekrümmten reflektierenden Fläche 52 in unterschiedliche Richtungen 62 und Raumwinkelbereiche gelenkt wird.

Bei der in Figur 2 gezeigten distalen Position der gekrümmte reflektierende Fläche 52 wird das von der Linse 36 kollimierte Beleuchtungslicht von einem Bereich der reflektierenden Fläche 52 reflektiert, deren Flächennormalen einen großen Winkel zur Längsachse 18 des Endoskops 10 einschließen. Entsprechend wird das Beleuchtungslicht in einen Raumwinkelbereich geworfen, der von der Richtung der Längsachse 18 des Endoskops 10 weit beabstandet ist.

Bei der in Figur 3 gezeigten Position der verschiebbaren gekrümmten reflektierenden Fläche 52 fällt das von der Linse 36 kollimierte Beleuchtungslicht auf einen Bereich der reflektierenden Fläche 52, in dem die lokalen Flächennormalen der reflektierenden Fläche 52 einen deutlich kleineren Winkel mit der Längsachse 18 des Endoskops 10 einschließen. Entsprechend wird das Beleuchtungslicht in einen Raumwinkelbereich geworfen, dessen Mitte nah an der Richtung der Längsachse 18 des Endoskops 10 liegt. Da die verschiebbare gekrümmte reflektierende Fläche 52 glatt ist, kann durch Verschieben der gekrümmten reflektierenden Fläche 52 jede beliebige Beleuchtungsrichtung 62 innerhalb eines vorbestimmten Intervalls eingestellt werden.

Figur 4 zeigt eine schematische Schnittdarstellung einer weiteren Ausführungsform des distalen Endes 11 eines Endoskops, beispielsweise des oben anhand der Figur 1 dargestellten Endoskops 10. Die Ausführungsform der Figur 4 ähnelt in einigen Merkmalen der Ausführungsform der Figuren 2 und 3. Die Ausführungsform der Figur 4 unterscheidet sich von der Ausführungsform der Figuren 2 und 3 insbesondere dadurch, dass die verschiebbare gekrümmte reflektierende Fläche 52 nicht konvex, sondern konkav ausgebildet ist.

In Figur 4 sind zwei verschiedene mögliche Positionen der verschiebbaren gekrümmten reflektierenden Fläche 52 dargestellt, eine in durchgezogener und eine in gestrichelter Linie. Bei der Position, in der die verschiebbare gekrümmte reflektierende Fläche 52 als durchgezogene Linie dargestellt ist, wird das von der Lichtaustrittsfläche 32 des Lichtleiters 30 ausgehende und von der Linse 36 kollimierte Beleuchtungslicht von der verschiebbaren gekrümmten reflektierenden Fläche 52 in eine mittlere Richtung bzw. eine Beleuchtungsrichtung 62 geworfen, die mit der Längsachse 18 des Endoskops 10 einen kleinen Winkel einschließt. Bei der Position der verschiebbaren gekrümmten reflektierenden Fläche, in der diese gestrichelt dargestellt ist, wird das von der Lichtaustrittsfläche 32 des Lichtleiters 30 ausgehende, von der Linse 36 kollimierte und von der verschiebbaren gekrümmten reflektierenden Fläche 52 reflektierte Beleuchtungslicht in einen Raumwinkelbereich und eine Richtung geworfen, die mit der Längsachse 18 des Endoskops 10 einen großen Winkel einschließt.

Aufgrund der konkaven Gestalt der verschiebbaren gekrümmten reflektierenden Fläche 52 weist der Beleuchtungs-Lichtkegel 60 bei beiden Positionen der verschiebbaren gekrümmten reflektierenden Fläche 52 eine Einschnürung 61 auf. Die Einschnürung 61 ist Figur 4 idealisiert punktförmig dargestellt ist. Tatsächlich weist die Einschnürung 61 schon aufgrund der Beugungsbegrenzung und der Ausdehnung der Lichtaustrittsfläche 32 des Lichtleiters 30 eine endliche Ausdehnung auf.

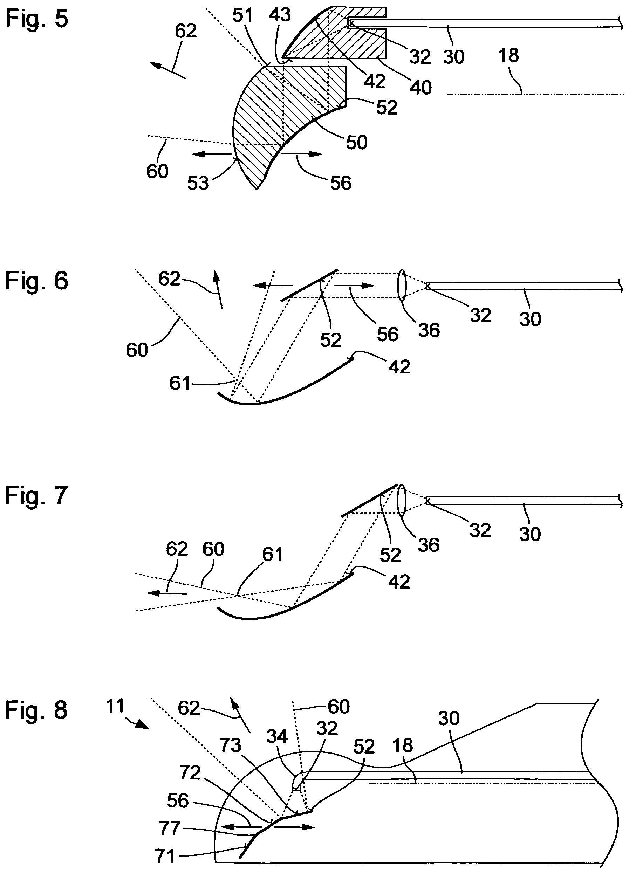

Figur 5 zeigt eine schematische Darstellung einer weiteren Ausführungsform des distalen Endes eines Endoskops, insbesondere des oben anhand der Figur 1 dargestellten Endoskops 10. In Gegensatz zu den Figuren 2 bis 4 sind in Figur 5 lediglich Einrichtungen gezeigt, die das Beleuchtungslicht leiten oder führen. Die Kontur des Schafts des Endoskops 10 und andere Einrichtungen sind in Figur 5 nicht gezeigt.

Bei den oben anhand der Figuren 2 bis 4 dargestellten Ausführungsformen sind die Flächennormalen der Lichtaustrittsflächen 32 der Lichtleiter 30 im Wesentlichen senkrecht zur Längsachse 18 des Endoskops 10. In Gegensatz dazu sind bei der Ausführungsform der Figur 5 der Lichtleiter im Wesentlichen gerade und die Flächennormale der Lichtaustrittsfläche 32 im Wesentlichen parallel zur Längsachse 18 des Endoskops 10. Das aus der Lichtaustrittsfläche 32 des Lichtleiters 30 austretende Beleuchtungslicht wird in einen feststehenden transparenten Körper 40 eingekoppelt. Insbesondere ist die Lichtaustrittsfläche 32 des Lichtleiters 30 mit dem transparenten Körper verklebt, verkittet oder verschweißt. Bei dem in Figur 5 gezeigten Beispiel ist das distale Ende des Lichtleiters 30 ferner in einer Bohrung des transparenten Körpers 40 angeordnet.

Der transparente Körper 40 weist eine reflektierende Fläche 42 auf. Die reflektierende Fläche reflektiert aufgrund von Totalreflexion oder aufgrund einer reflektierenden Beschichtung. Die reflektierende Fläche 42 ist ausgebildet und angeordnet, um das von der Lichtaustrittsfläche 32 des Lichtleiters 30 ausgehende Beleuchtungslicht in eine Richtung im Wesentlichen senkrecht zur Längsachse 18 des Endoskops 10 zu lenken. Insbesondere ist die reflektierende Fläche 42 des transparenten Körpers 40 gekrümmt, um das Beleuchtungslicht zu kollimieren. Dazu weist die reflektierende Fläche 42 des transparenten Körpers 40 insbesondere die Gestalt eines Ausschnitts eines Rotationsparaboloids auf.

Der transparente Körper 40 weist ferner eine Lichtaustrittsfläche 43 auf, durch die das von der Lichtaustrittsfläche 32 des Lichtleiters 30 ausgehende und an der reflektierenden Fläche 42 des transparenten Körpers 40 reflektierte Beleuchtungslicht in einer Richtung im Wesentlichen senkrecht zur Längsachse 18 des Endoskops 10 austritt. Bei dem dargestellten Beispiel ist die Lichtaustrittsfläche 43 des feststehenden transparenten Köpers 40 eben und weist eine Flächennormale auf, die im Wesentlichen senkrecht zur Längsachse 18 des Endoskops 10 ist.

Gegenüber der Lichtaustrittsfläche 43 des feststehenden transparenten Körpers 40 ist eine Lichteintrittsfläche 51 eines verschiebbaren transparenten Körpers 50 angeordnet. Die Lichtaustrittsfläche 43 des feststehenden transparenten Körpers und die Lichteintrittsfläche 51 des verschiebbaren transparenten Körpers 50 sind insbesondere parallel zueinander und jeweils eben. Der verschiebbare transparente Körper 50 weist ferner eine gekrümmte reflektierende Fläche 52 und eine Lichtaustrittsfläche 53, die bei dem dargestellten Beispiel ebenfalls gekrümmt ist, auf.

Die gekrümmte reflektierende Fläche 52 des verschiebbaren transparenten Körpers 50 reflektiert Beleuchtungslicht beispielsweise aufgrund von Totalreflexion oder aufgrund einer reflektierenden Beschichtung. Die gekrümmte reflektierende Fläche 52 ist an einem zumindest bezogen auf die in Figur 5 dargestellte Schnittebene konkaven Abschnitt der Oberfläche des verschiebbaren transparenten Körpers 50 angeordnet. Bezogen auf den Raumbereich, in dem sich Beleuchtungslicht ausbreitet, ist die reflektierende Fläche 52 konvex gekrümmt.

Der transparente Körper 50 ist in einer durch Pfeile 56 angedeuteten Richtung parallel oder im Wesentlichen parallel zur Längsachse 18 des Endoskops 10 verschiebbar. Ähnlich wie bei den Ausführungsformen der Figuren 2 und 3 wird das von der verschiebbaren reflektierenden Fläche 52 reflektierte Beleuchtungslicht abhängig von der Position des transparenten Körpers 50 und der gekrümmten reflektierenden Fläche 52 in unterschiedliche Raumwinkelbereiche geworfen. Für die dargestellte mittlere Position des verschiebbaren transparenten Körpers 50 ist die Ausbreitung des von der Lichtaustrittsfläche 32 des Lichtleiters 30 ausgehenden Beleuchtungslichts ähnlich wie bei den Figuren 2 bis 4 qualitativ durch Konturen eines Beleuchtungs-Lichtkegels 60 angedeutet.

Die Figuren 6 und 7 zeigen schematische Darstellungen einer weiteren Ausführungsform des distalen Endes eines Endoskops, beispielsweise des oben anhand der Figur 1 dargestellten Endoskops 10. Ähnlich wie bei Figur 5 sind lediglich optische Einrichtungen im Beleuchtungs-Strahlengang dargestellt. Ebenso wie bei den Figuren 2 bis 5 sind keine optischen Einrichtungen im Beobachtungs-Strahlengang oder mechanische Einrichtungen zum Führen oder Halten der optischen Einrichtungen dargestellt. Die dargestellte Schnittebene ist parallel zur Längsachse 18 des Endoskops 10.

Ähnlich wie bei der Ausführungsform der Figur 5 weist der Lichtleiter 30 nahe seiner Lichtaustrittsfläche 32 keine Krümmung auf. Ähnlich wie bei den Ausführungsformen der Figuren 2 bis 4 ist eine Linse 36 zum Kollimieren des von der Lichtaustrittsfläche 32 des Lichtleiters 30 ausgehenden Beleuchtungslichts vorgesehen. Ferner sind eine gekrümmte feststehende reflektierende Fläche 42 und eine verschiebbare ebene reflektierende Fläche 52 vorgesehen. Die verschiebbare ebene reflektierende Fläche 52 und die feststehende gekrümmte reflektierende Fläche 42 sind so angeordnet, dass von der Lichtaustrittsfläche 32 des Lichtleiters 30 ausgehendes und von der Linse 36 kollimiertes Beleuchtungslicht zunächst auf die verschiebbare ebene reflektierende Fläche 52 fällt und von dieser reflektiert wird. Das von der verschiebbaren ebenen reflektierenden Fläche 52 reflektierte Beleuchtungslicht fällt auf die feststehende gekrümmte reflektierende Fläche 42 und wird von dieser reflektiert.

Die verschiebbare ebene reflektierende Fläche 52 ist in einer Richtung 56 parallel oder im Wesentlichen parallel zur Längsachse 18 des Endoskops 10 verschiebbar. Abhängig von der Position der verschiebbaren ebenen reflektierenden Fläche 52 fällt von dieser reflektiertes Beleuchtungslicht auf unterschiedliche Bereiche der feststehenden gekrümmten reflektierenden Fläche 42. Aufgrund der Krümmung der feststehenden reflektierenden Fläche 42 wird das Beleuchtungslicht abhängig von der Position der verschiebbaren ebenen reflektierenden Fläche 52 von der feststehenden gekrümmten reflektierenden Fläche 42 in unterschiedliche Raumwinkelbereiche geworfen. In den Figuren 6 und 7 sind zwei verschiedene Positionen der verschiebbaren ebenen reflektierenden Fläche 52 gezeigt. Ferner sind durch Konturen je eines Beleuchtungs-Lichtkegels 60 die Raumwinkelbereiche angedeutet, in die das Beleuchtungslicht geworfen wird. Ähnlich wie bei der Ausführungsform der Figur 4 weist der Beleuchtungs-Lichtkegel 60 aufgrund der konkaven Gestalt der feststehenden gekrümmten reflektierenden Fläche 42 eine Einschnürung 61 auf.

Figur 8 zeigt eine schematische Darstellung einer weiteren Ausführungsform des distalen Endes eines Endoskops, insbesondere des oben anhand der Figur 1 dargestellten Endoskops 10. Die Ausfiihrungsform der Figur 8 ähnelt in einigen Merkmalen den Ausführungsformen der Figuren 2 bis 7, insbesondere der Ausführungsform der Figuren 2 und 3. Insbesondere ist eine in einer Richtung 56 verschiebbare reflektierende Fläche 52 vorgesehen. Abhängig von der Position der verschiebbaren reflektierenden Fläche 52 wird aus einer Lichtaustrittsfläche 32 eines Lichtleiters 30 austretendes Beleuchtungslicht in unterschiedliche Richtungen 62 geworfen.

Die Ausführungsform der Figur 8 unterscheidet sich von der Ausführungsform der Figuren 2 und 3 insbesondere dadurch, dass die verschiebbare reflektierende Fläche 52 nicht glatt ist. Die reflektierende Fläche 52 umfasst mehrere ebene oder im Wesentlichen ebene Abschnitte 71, 72, 73. In dem in Figur 8 dargestellten Schnitt grenzen die Abschnitte 71, 72, 73 der verschiebbaren reflektierenden Fläche 52 aneinander. Zwischen den Abschnitten 71, 72, 73 weist die verschiebbare reflektierende Fläche 52 jeweils einen Knick 77 auf.

Die Abschnitte 71, 72, 73 der verschiebbaren reflektierenden Fläche 52 können in einer Richtung senkrecht zur Zeichenebene der Figur 8 gekrümmt sein. Insbesondere sind die Abschnitte 71, 72, 73 in der genannten Richtung konkav gekrümmt, um den Querschnitt des Beleuchtungslichtbündels zu verringern. Alternativ können die Abschnitte 71, 72, 73 der verschiebbaren reflektierenden Fläche 52 jeweils eine kuppelförmig oder eine sattelförmig Gestalt aufweisen. Abweichend von der Darstellung in Figur 8 können zwischen den Abschnitten 71, 72, 73 der verschiebbaren reflektierenden Fläche 52 Stufen und/oder Lücken vorliegen.

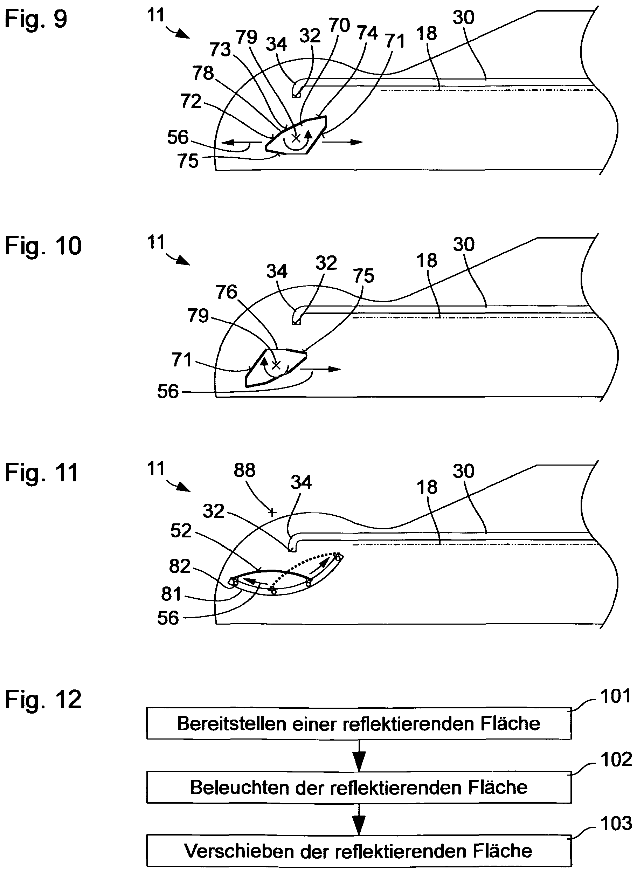

Die Figuren 9 und 10 zeigen schematische Darstellungen einer weiteren Ausuhrungsform des distalen Endes eines Endoskops, insbesondere des oben anhand der Figur 1 dargestellten Endoskops 10. Die Ausführungsform der Figuren 9 und 10 ähnelt in einigen Merkmalen den Ausführungsformen der Figuren 2 bis 8, insbesondere der Ausführungsform der Figur 8. Insbesondere ist eine in einer Richtung 56 verschiebbare reflektierende Fläche vorgesehen, die mehrere Abschnitte 71, 72, 73, 74, 75 aufweist.

Im Gegensatz zu den Darstellungen der Figuren 2 bis 8 sind Beleuchtungs-Lichtkegel 60 und Beleuchtungsrichtung 62 in den Figuren 9 und 10 nicht gezeigt, um die Darstellung nicht zu überfrachten. Für Fachleute ist jedoch leicht erkennbar, in welche Richtungen die Abschnitte 71, 72, 73, 74, 75 der verschiebbaren reflektierenden Fläche Beleuchtungslicht, das aus der Lichtaustrittfläche 32 des Lichtleiters 30 austritt, reflektieren.

Die Abschnitte 71, 72, 73, 74, 75 der verschiebbaren reflektierenden Fläche sind an einem Spiegelträger 70 angeordnet. Die Ausführungsform der Figuren 9 und 10 unterscheidet sich von der Ausführungsform der Figur 8 insbesondere dadurch, dass der Spiegelträger 70 mit den Abschnitten 71, 72, 73, 74, 75 der reflektierenden Fläche in der Richtung 56 linear verschiebbar und um eine Achse 79 schwenkbar bzw. rotierbar ist. Insbesondere ist der Spiegelträger 70 vorgesehen und ausgebildet, um in zwei Orientierungen verwendet zu werden. In Figur 9 und Figur 10 ist jeweils eine der beiden Orientierungen gezeigt.

In der in Figur 9 dargestellten Orientierung kann der Spiegelträger 70 mit der reflektierenden Fläche 52 in der Richtung 56 verschoben werden. Bei der in Figur 9 dargestellten Position des Spiegelträgers 70 fällt von der Lichtaustrittsfläche 34 des Lichtleiters 30 ausgehendes Beleuchtungslicht überwiegend oder ausschließlich auf den dritten Abschnitt 73 der reflektierenden Fläche 52. In anderen, allein durch lineare Verschiebung erreichbaren Positionen des Spiegelträgers 70 fällt das Beleuchtungslicht überwiegend oder ausschließlich auf den zweiten Abschnitt 72 oder auf den vierten Abschnitt 74 der reflektierenden Fläche 52.

In der in Figur 10 dargestellten Orientierung und Position des Spiegelträgers 70 fällt von der Lichtaustrittsfläche 32 des Lichtleiters 30 ausgehende Beleuchtungslicht überwiegend oder ausschließlich auf den fünften Abschnitt 75 der reflektierenden Fläche 52. In der in Figur 10 dargestellten Orientierung kann der Spiegelträger 70 in der Richtung 56 so weit verschoben werden, dass von der Lichtaustrittfläche 32 ausgehendes Beleuchtungslicht überwiegend oder ausschließlich auf den ersten Abschnitt 71 der reflektierenden Fläche fällt.

In Zusammenschau der Figuren 9 und 10 ist erkennbar, dass die Abschnitte 71, 72, 73, 74, 75 in der jeweils vorgesehenen Orientierung des Spiegelträgers 70 unterschiedliche Neigungen aufweisen bzw. unterschiedliche Winkel mit der Längsachse 18 des Endoskops 10 einschließen. Die Flächennormale des ersten Abschnitts 71 der reflektierenden Fläche schließt bei der in Figur 10 dargestellten Orientierung des Spiegelträgers 70 mit der Längsachse 18 einen verhältnismäßig kleinen Winkel ein. Die Flächennormale des fünften Abschnitts 75 der reflektierenden Fläche schließt bei der in Figur 10 gezeigten Orientierung des Spiegelträgers 70 mit der Längsachse 18 einen verhältnismäßig großen Winkel ein. Die Winkel, die die Flächennormalen des zweiten Abschnitts 72, des dritten Abschnitts 73 und des vierten Abschnitts 74 mit der Längsachse 18 des Endoskops bei der in Figur 9 gezeigten Orientierung einschließen, liegen zwischen den anderen beiden Winkeln. Indem der Spiegelträger 70 in eine der beiden in den Figuren 9 und 10 dargestellten Orientierungen rotiert und in der Richtung 56 verschoben wird können somit bei dem dargestellten Beispiel fünf verschiedene Beleuchtungsrichtungen eingestellt werden.

Bei dem in den Figuren 9 und 10 dargestellten Beispiel grenzen nicht alle nächst benachbarte Abschnitte 71, 72, 73, 74, 75 der reflektierenden Fläche unmittelbar aneinander. Vielmehr liegt beispielsweise zwischen dem ersten Abschnitt 71 und dem fünften Abschnitt 75 der reflektierenden Fläche 52 eine Lücke 76 vor, in der der Spiegelträger 70 insbesondere nicht reflektierend beschichtet ist. Alternativ kann der Spiegelträger 70 auch in der Lücke 76 eine reflektierende Beschichtung aufweisen. Zwischen dem zweiten Abschnitt 72, dem dritten Abschnitt 73 und dem vierten Abschnitt 74 weist die reflektierende Fläche jeweils lediglich einen Knick 78 auf.

Abweichend von den Darstellungen in den Figuren 9 und 10 können die Abschnitte 71, 72, 73, 74, 75 der reflektierenden Fläche, ähnlich wie in Zusammenhang mit der Ausführungsform der Figur 8 beschrieben, gekrümmt sein. Ferner kann abweichend von den Darstellungen in den Figuren 9 und 10 in jeder Orientierung des Spiegelträgers 70 lediglich ein glatter Abschnitt der reflektierenden Fläche vorgesehen sein.

Figur 11 zeigt eine schematische Darstellung einer weiteren Ausführungsform des distalen Endes eines Endoskops, insbesondere des oben anhand der Figur 1 dargestellten Endoskops 10. Die Ausführungsform der Figur 11 ähnelt in einigen Merkmalen den Ausführungsformen der Figuren 2 bis 7, insbesondere der Ausführungsform der Figuren 2 und 3. Insbesondere ist eine glatte, gekrümmte und verschiebbare reflektierende Fläche 52 vorgesehen.

Die Ausführungsform der Figur 11 unterscheidet sich von der Ausführungsform der Figuren 2 und 3 insbesondere dadurch, dass die verschiebbare reflektierende Fläche 52 entlang eines gekrümmten Pfads verschiebbar ist. Der gekrümmte Pfad ist durch eine gekrümmte Schiene 81 vorgegeben. Die reflektierende Fläche 52 ist mit mehreren Führungselementen 82 mechanisch gekoppelt, insbesondere starr verbunden. Die führungselemente 82 sind in oder an der Schiene 81 geführt. Die Schiene 81 wird beispielsweise durch eine Nut oder durch einen Steg gebildet. Insbesondere sind zwei parallele Schienen 81 an gegenüberliegenden Seiten der reflektierenden Fläche 52 vorgesehen.

In Figur 11 sind zwei Positionen der reflektierenden Fläche 52 dargestellt, in denen die der Lichtaustrittsfläche 32 des Lichtleiters 30 jeweils gegenüberliegenden Bereiche der reflektierenden Fläche 52 unterschiedlich orientiert sind. Durch verschieben der reflektierenden Fläche entlang der Schiene 81 können somit ähnlich wie bei der Ausführungsform der Figuren 2 und 3 verschiedene Beleuchtungsrichtungen eingestellt werden.

Bei dem dargestellten Beispiel weist die Schiene 81 eine kreisbogenförmige Gestalt auf. Der Krümmungsmittelpunkt 88 der Schiene 81 liegt außerhalb des Schafts des Endoskops. Alternativ weist die Schiene 81 eine variierende Krümmung auf, wobei der Krümmungsmittelpunkt insbesondere zumindest für einen Abschnitt der Schiene 81 außerhalb des Schafts des Endoskops liegt. Ferner kann für jeden Gleitstein 82 eine eigene gerade oder gekrümmte Schiene 81 vorgesehen sein.

Schienen ähnlich der in Figur 11 dargestellten Schiene 81, jedoch insbesondere in gerader Ausführung, können auch bei den Ausführungsformen der Figuren 2 bis 10 verwendet werden, um die reflektierende Fläche zu führen.

Einige Merkmale der oben anhand der Figuren 2 bis 11 dargestellten Ausführungsformen sind abweichend von den Ausführungsformen kombinierbar. Beispielsweise können auch die verschiebbaren gekrümmten reflektierenden Flächen 52 der Ausführungsformen der Figuren 2 bis 4, 8 und 11 sowie die verschiebbare ebene reflektierende Fläche 42 und/oder die feststehende gekrümmte reflektierende Fläche 52 der Ausführungsform der Figuren 6 und 7 ähnlich wie bei der Ausführungsform der Figur 5 an transparenten Körpern angeordnet sein. Auch bei der Ausführungsform der Figur 5 können die feststehende gekrümmte reflektierende Fläche 52 und/oder die verschiebbare gekrümmte reflektierende Fläche 42 ähnlich wie die reflektierenden Flächen der Ausführungsformen der Figuren 2 bis 4 und 6 bis 11 als Spiegel ausgeführt sein, bei denen das Beleuchtungslicht an einer offenliegenden reflektierenden Schicht reflektiert wird oder vor und nach der Reflexion lediglich durch eine dünne transparente Schicht läuft.

Ferner kann bei allen Ausführungsformen eine Kollimation des aus der Lichtaustrittsfläche 32 des Lichtleiters 30 austretenden Beleuchtungslichts durch andere Maßnahmen bewirkt werden. Alternativ kann auf eine Kollimation des Beleuchtungslichts ganz verzichtet werden. Ferner kann das Beleuchtungslicht gebündelt bzw. der Querschnitt des Beleuchtungs-Lichtbündels in der Nähe der reflektierenden Fläche 52 bzw. 42 reduziert werden. Insbesondere kann das Beleuchtungs-Lichtbündel an oder nahe der feststehenden reflektierenden Fläche 42 und/oder an oder nahe der verschiebbaren reflektierenden Fläche 52 eine Einschnürung bzw. eine Taille aufweisen.

Bei allen Ausführungsformen kann die gekrümmte reflektierende Fläche 52 bzw. 42 in einer Richtung senkrecht zur dargestellten Zeichenebene ebenfalls gekrümmt sein. Insbesondere weist die gekrümmte reflektierende Fläche 52 bei den Ausführungsformen der Figuren 2, 3 und 5 jeweils eine negative Gaußsche Krümmung K bzw. die Gestalt einer Sattelfläche auf. Insbesondere weist die gekrümmte reflektierende Flächen 52 bzw. 42 bei den Ausführungsformen der Figuren 4, 6, 7 und 11 jeweils eine positive Gaußsche Krümmung K > 0 bzw. eine kuppelförmige Gestalt auf. Durch eine derartige Ausgestaltung der gekrümmten reflektierenden Fläche 52 bzw. 42 kann die Ausdehnung des BeleuchtungsLichtbündels 60 unmittelbar lichtstromabwärts der gekrümmten reflektierenden Fläche 52 bzw. 42 in einer Richtung senkrecht zu den Zeichenebenen der Figuren 2 bis 11 reduziert werden, um Bauraum im Beleuchtungs-Strahlengang einzusparen.

Bei den Ausführungsform der Figuren 2 bis 4 ist die Lichtaustrittsfläche der Linse 36 die der gekrümmten reflektierenden Fläche 52 lichtstromaufwärts nächste Lichtaustrittsfläche. Anders ausgedrückt ist die Lichtaustrittsfläche der Linse 36 die letzte Lichtaustrittsfläche, durch die Beleuchtungslicht hindurchtritt bevor es auf die verschiebbare reflektierende Fläche 52 fällt. Bei den Ausführungsformen der Figuren 2 bis 4 sind die Flächennormale der Lichtaustrittsfläche 32 des Lichtleiters 30, die mittlere Flächennormale der Lichtaustrittsfläche der Linse 36 und die Symmetrieachse der Linse 36 senkrecht oder im Wesentlichen senkrecht zur Längsachse 18 des Endoskops 10.

Bei der Ausführungsform der Figur 5 ist die Lichtaustrittsfläche 43 des feststehenden transparenten Körpers 40 die der gekrümmten reflektierenden Fläche 52 lichtstromaufwärts nächste Lichtaustrittsfläche. Anders ausgedrückt ist die Lichtaustrittsfläche 43 des feststehenden transparenten Körpers 40 die letzte Lichtaustrittsfläche, durch die Beleuchtungslicht hindurchtritt bevor es auf die verschiebbare reflektierende Fläche 52 fällt. Bei der Ausführungsform der Figur 5 ist die Flächennormale der Lichtaustrittsfläche 43 des feststehenden transparenten Körpers 40 senkrecht oder im Wesentlichen senkrecht zur Längsachse 18 des Endoskops 10.

Alternativ können bei den Ausführungsformen der Figuren 2 bis 5 und 8 bis 11 die Ausbreitungsrichtung des Beleuchtungslichts und/oder die Flächennormalen der Lichtaustrittsfläche 32 des Lichtleiters 30 bzw. der Lichtaustrittsfläche 43 des feststehenden transparenten Körpers 40 mit der Längsachse 18 des Endoskops 10 einen Winkel einschließen, der kleiner als 90 Grad ist, ähnlich der Ausführungsform der Figuren 6 und 7.

Bei allen Ausführungsformen kann anstelle eines Lichtleiters 30 eine Leuchtdiode oder eine andere Lichtquelle mit einer Lichtaustrittsfläche am distalen Ende 11 des Endoskops 10 vorgesehen sein. Bei allen Ausführungsformen der Figuren 2 bis 11 kann das Endoskop 10 von der Darstellung anhand der Figur 1 abweichende Eigenschaften und Merkmale aufweisen.

Figur 12 zeigt ein schematisches Flussdiagramm eines Verfahrens zum Einstellen der Beleuchtungsrichtung eines Endoskops. Das Verfahren ist insbesondere mit den Endoskopen ausführbar, die oben anhand der Figuren 2 bis 11 dargestellt sind. Alternativ ist das Verfahren jedoch auch mit Endoskopen ausführbar, die von den Ausführungsformen der Figuren 2 bis 11 abweichende Eigenschaften aufweisen. Nachfolgend werden trotzdem beispielhaft Bezugszeichen aus den Figuren 2 bis 11 verwendet.

Bei einem ersten Schritt 101 wird eine reflektierende Fläche 52 zum Reflektieren von Beleuchtungslicht zur Beleuchtung eines mittels des Endoskops 10 beobachteten Gegenstands bereitgestellt. Bei einem zweiten Schritt 102 wird die reflektierende Fläche 52 mit Beleuchtungslicht beleuchtet, das von einer Lichtaustrittsfläche 32, insbesondere von einer Lichtaustrittsfläche 32, 43 eines Lichtleiters 30, eines transparenten Körpers 40 oder einer Lichtquelle ausgeht. Das Beleuchtungslicht fällt insbesondere aus einer Richtung auf die reflektierende Fläche 52, die nicht parallel zur Längsachse 18 des Endoskops und nicht parallel zur Schwenkachse der Blickrichtung und nicht parallel zur Schwenkachse der Beleuchtungsrichtung ist.

Bei einem dritten Schritt 103 wird die reflektierende Fläche 52 verschoben, um die Beleuchtungsrichtung einzustellen. Dabei wird die reflektierende Fläche insbesondere durch eine gerade oder gekrümmte Linearführung 81, 82 geführt.

### Bezugszeichen

- 10: Endoskop
- 11: distales Ende des Endoskops 10
- 12: proximales Ende des Endoskops 10
- 14: Schaft des Endoskops 10
- 15: erste Kupplung
- 16: zweite Kupplung
- 18: Längsachse des Endoskops 10
- 20: Oberfläche eines Fensterbauteils
- 21: erste extreme Blickrichtung (0 Grad)
- 22: zweite extreme Blickrichtung (120 Grad)
- 28: Schwenkachse der Blickrichtung
- 29: Winkelbereich der Blickrichtungen
- 30: Lichtwellenleiter
- 32: Lichtaustrittsfläche des Lichtwellenleiters 30
- 34: Krümmung des Lichtwellenleiters nahe seiner Lichtaustrittsfläche 32
- 36: Linse

- 40: feststehender transparenter Körper
- 41: Lichteintrittsfläche des feststehenden transparenten Körpers 40
- 42: feststehende reflektierende Fläche
- 43: Lichtaustrittsfläche des feststehenden transparenten Körpers 40
- 48: photolumineszierender Körper

- 50: verschiebbarer transparenter Körper
- 51: Lichteintrittsfläche des verschiebbaren transparenten Körpers 50
- 52: verschiebbare reflektierende Fläche
- 53: Lichtaustrittsfläche des verschiebbaren transparenten Körpers 50
- 56: Richtung, in der die reflektierende Fläche 52 verschiebbar ist

- 60: Beleuchtungs-Lichtkegel
- 61: Einschnürung des Beleuchtungs-Lichtkegels 60
- 62: Beleuchtungsrichtung

- 70: Spiegelträger
- 71: erster Abschnitt der reflektierenden Fläche 52
- 72: zweiter Abschnitt der reflektierenden Fläche 52
- 73: dritter Abschnitt der reflektierenden Fläche 52
- 74: vierter Abschnitt der reflektierenden Fläche 52
- 75: fünfter Abschnitt der reflektierenden Fläche 52
- 76: Lücke zwischen dem fünften Abschnitt 75 und dem ersten Abschnitt 71
- 77: Knick zwischen dem ersten Abschnitt 71 und dem zweiten Abschnitt 72
- 78: Knick zwischen dem zweiten Abschnitt 72 und dem dritten Abschnitt 73
- 79: Achse, um die die reflektierende Fläche 52 schwenkbar ist

- 81: Schiene
- 82: Führungselement
- 88: Krümmungsmittelpunkt der Schiene 81

- 101: erster Schritt
- 102: zweiter Schritt
- 103: dritter Schritt

## Patentansprüche

1. **Endoskop** (10) **mit einstellbarer Beleuchtungsrichtung** (60, 62), mit:
einem sich durch einen Schaft (14) bis zum distalen Ende (11) des Endoskops (10) erstreckenden Lichtleiter (30) zur Übertragung von Beleuchtungslicht, einer **reflektierenden Fläche** (52) zum Reflektieren von Beleuchtungslicht zur Beleuchtung eines mittels des Endoskops (10) beobachteten Gegenstands,
wobei die reflektierende Fläche (52) **verschiebbar** ist, um die Beleuchtungsrichtung (60, 62) einzustellen, wobei eine Schnittlinie der verschiebbaren reflektierenden Fläche (52) mit einer Ebene, die zu zwei einstellbaren Beleuchtungsrichtungen (60, 62) des Endoskops (10) parallel ist, konvex **gekrümmt** ist, derart, dass die verschiebbare reflektierende Fläche (52), bezogen auf den Raumbereich, in dem sich das Beleuchtungslicht ausbreitet, konvex gekrümmt ist, ferner mit:
einer **Linearführung** (81, 82) zum Führen der verschiebbaren reflektierenden Fläche (52) entlang eines geraden oder gekrümmten Pfads, ferner mit:
einer Lichtaustrittsfläche (43) zum Bestrahlen der verschiebbaren reflektierenden Fläche (52) mit Beleuchtungslicht, wobei eine Flächennormale der Lichtaustrittsfläche (43) nicht parallel zu einer Längsachse (18) des Endoskops (10) und nicht parallel zur einer Schwenkachse (28) der Beleuchtungsrichtung oder zu einer Schwenkachse der Blickrichtung ist, wobei
die Lichtaustrittsfläche (43) eine Lichtaustrittsfläche (43) eines transparenten Körpers (40) zum Lenken oder Umlenken des Beleuchtungslichts ist,
**dadurch gekennzeichnet, dass**
der transparente Körper (40) eine Bohrung aufweist, in dem das distale Ende des Lichtleiters (30) angeordnet ist.

2. Endoskop (10) nach dem vorangehenden Anspruch, bei dem die reflektierende Fläche (52) in einer Richtung (56) **verschiebbar** ist, die mit der Längsachse (18) des Endoskops (10) einen Winkel von **nicht mehr als 45 Grad** einschließt.

3. Endoskop (10) nach einem der vorangehenden Ansprüche, bei dem die reflektierende Fläche (52) entlang eines **gerade Pfads** (58) verschiebbar ist.

4. Endoskop (10) nach einem der vorangehenden Ansprüche, bei dem die reflektierende Fläche (52) entlang eines **gekrümmten Pfads** (56) verschiebbar ist, der einen lokalen Krümmungsmittelpunkt aufweist, der zumindest entweder vom Ort der reflektierenden Fläche abhängig ist oder außerhalb des Schafts (14) des Endoskops (10) liegt.

5. Endoskop (10) nach einem der vorangehenden Ansprüche, wobei das Endoskop (10) ausgebildet ist, um **Beleuchtungslicht** aus einer Richtung auf die verschiebbare reflektierende Fläche zu werfen, die **nicht parallel** zur Längsachse (18) des Endoskops (10) ist.

6. Endoskop (10) nach einem der vorangehenden Ansprüche, bei dem die verschiebbare reflektierende Fläche (52) einen ersten Abschnitt (54) und einen zweiten Abschnitt (55) umfasst, wobei zumindest entweder der erste Abschnitt (71) und der zweite Abschnitt (72) **nicht glatt** in einander übergehen oder die Schnittlinien des ersten Abschnitts und des zweiten Abschnitts mit einer Ebene, die zu zwei einstellbaren Beleuchtungsrichtungen (21, 22) des Endoskops (10) parallel ist, entgegengesetzt gekrümmt sind.

## Claims

1. Endoscope (10) having an adjustable illumination direction (60, 62), having:
a light waveguide (30) which extends through a shaft (14) to the distal end (11) of the endoscope (10) and which serves for transmitting illumination light,
a reflective surface (52) for reflecting illumination light for the illumination of an object observed by means of the endoscope (10),
the reflective surface (52) being displaceable in order to adjust the illumination direction (60, 62),
a line of intersection of the displaceable reflective surface (52) with a plane which is parallel to two adjustable illumination directions (60, 62) of the endoscope (10) being convexly curved such that the displaceable reflective surface (52) is convexly curved relative to the spatial region in which the illumination light propagates,
furthermore having:
a linear guide (81, 82) for guiding the displaceable reflective surface (52) along a straight or curved path,
furthermore having:
a light exit surface (43) for exposure of the displaceable reflective surface (52) to illumination light, a surface normal of the light exit surface (43) being not parallel to a longitudinal axis (18) of the endoscope (10) and not parallel to a pivot axis (28) of the illumination direction or to a pivot axis of the viewing direction, wherein the light exit surface (43) is a light exit surface (43) of a transparent body (40) for directing or deviating the illumination light, **characterized in that** the transparent body (40) has a bore in which the distal end of the light waveguide (30) is arranged.

2. Endoscope (10) according to the preceding claim, wherein the reflective surface (52) is displaceable in a direction (56) which makes an angle of not more than 45 degrees with the longitudinal axis (18) of the endoscope (10).

3. Endoscope (10) according to one of the preceding claims, wherein the reflective surface (52) is displaceable along a straight path (58).

4. Endoscope (10) according to one of the preceding claims, wherein the reflective surface (52) is displaceable along a curved path (56) which has a local center of curvature that at least either is dependent on the position of the reflective surface or lies outside the shaft (14) of the endoscope (10).

5. Endoscope (10) according to one of the preceding claims, wherein the endoscope (10) is formed in order to shine illumination light onto the displaceable reflective surface from a direction which is not parallel to the longitudinal axis (18) of the endoscope (10).

6. Endoscope (10) according to one of the preceding claims, wherein the displaceable reflective surface (52) comprises a first section (54) and a second section (55), with at least either the first section (71) and the second section (72) merging non-smoothly into one another or the section lines of the first section and the second section being oppositely curved with a plane which is parallel to two adjustable illumination directions (21, 22) of the endoscope (10).

## Revendications

1. Endoscope (10) ayant une direction d'éclairage réglable (60, 62), comprenant :
un guide de lumière (30) destiné à la transmission de la lumière d'éclairage, qui s'étend à travers une tige (14) jusqu'à une extrémité distale (11) de l'endoscope (10),
une surface réfléchissante (52) destinée à réfléchir la lumière d'éclairage en vue d'éclairer un objet observé au moyen de l'endoscope (10),
la surface réfléchissante (52) étant coulissante afin de régler la direction d'éclairage (60, 62),
une ligne d'intersection de la surface réfléchissante (52) coulissante avec un plan qui est parallèle aux deux directions d'éclairage réglables (60, 62) de l'endoscope (10) étant curviligne, de telle sorte que la surface réfléchissante (52) coulissante est de courbure convexe par rapport à la zone de l'espace dans laquelle se propage la lumière d'éclairage, comprenant en outre :
un guide linéaire (81, 82) destiné à guider la surface réfléchissante (52) coulissante le long d'un trajet droit ou curviligne, comprenant en outre :
une surface de sortie de lumière (43) destinée à l'irradiation de la surface réfléchissante (52) coulissante avec de la lumière d'éclairage, une normale à la surface de sortie de lumière (43) n'étant pas parallèle à un axe longitudinal (18) de l'endoscope (10) et pas parallèle à un axe de pivotement (28) de la direction d'éclairage ou à un axe de pivotement de la direction d'observation,
la surface de sortie de lumière (43) étant une surface de sortie de lumière (43) d'un corps transparent (40) destiné à diriger ou à dévier la lumière d'éclairage,
**caractérisé en ce que** le corps transparent (40) possède un corps dans lequel est disposée l'extrémité distale du guide de lumière (30).

2. Endoscope (10) selon la revendication précédente, dans lequel la surface réfléchissante (52) peut coulisser dans une direction (56) qui forme un angle maximum de 45 degrés avec l'axe longitudinal (18) de l'endoscope (10).

3. Endoscope (10) selon l'une des revendications précédentes, dans lequel la surface réfléchissante (52) peut coulisser le long d'un trajet rectiligne (58).

4. Endoscope (10) selon l'une des revendications précédentes, dans lequel la surface réfléchissante (52) peut coulisser le long d'un trajet curviligne (56), lequel possède un point central de courbure local qui est soit dépendant au moins de l'endroit de la surface réfléchissante, soit se trouve en dehors de la tige (14) de l'endoscope (10).

5. Endoscope (10) selon l'une des revendications précédentes, l'endoscope (10) étant configuré pour projeter la lumière d'éclairage sur la surface réfléchissante coulissante dans une direction qui n'est pas parallèle à l'axe longitudinal (18) de l'endoscope (10).

6. Endoscope (10) selon l'une des revendications précédentes, avec lequel la surface réfléchissante (52) coulissante comporte une première portion (54) et une deuxième portion (55), endoscope avec lequel soit au moins la première portion (71) et la deuxième portion (72) s'entremêlent de manière non glissante, soit les lignes d'intersection de la première portion et de la deuxième portion présentent des courbures opposées par rapport à un plan qui est parallèle aux deux directions d'éclairage (21, 22) réglables de l'endoscope (10).
